(19) Europäisches Patentamt
European Patent Office
Office européen des brevets

(11) **EP 3 047 798 B1**

(12) **FASCICULE DE BREVET EUROPEEN**

(45) Date de publication et mention
de la délivrance du brevet:
**06.09.2023 Bulletin 2023/36**

(21) Numéro de dépôt: **16157853.9**

(22) Date de dépôt: **08.06.2011**

(51) Classification Internationale des Brevets (IPC):
**A61B 5/11** *(2006.01)* **G01C 22/00** *(2006.01)*

(52) Classification Coopérative des Brevets (CPC):
**A61B 5/1038; A61B 5/112; A61B 5/1122;**
**A61B 5/6831; G01C 22/006;** A61B 2562/0219;
G06F 2218/00

(54) **DISPOSITIF PORTABLE INTÉGRÉ ET PROCÉDÉ METTANT EN OEUVRE UN ACCÉLÉROMÈTRE POUR ANALYSER DES PARAMÈTRES BIOMÉCANIQUES DE LA FOULÉE**

INTEGRIERTES TRAGBARES GERÄT UND VERFAHREN ZUR ANWENDUNG EINES BESCHLEUNIGUNGSMESSERS ZUR ANALYSE DER BIOCHEMISCHEN PARAMETER VON SCHRITTEN

INTEGRATED PORTABLE DEVICE AND METHOD USING AN ACCELEROMETER TO ANALYSE BIOMECHANICAL PARAMETERS OF THE STRIDE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.06.2010 CH 9732010**

(43) Date de publication de la demande:
**27.07.2016 Bulletin 2016/30**

(62) Numéro(s) de document de la (des) demande(s) initiale(s) en application de l'article 76 CBE:
**11725708.9 / 2 582 295**

(73) Titulaire: **Myotest SA**
**1950 Sion (CH)**

(72) Inventeurs:
• **Flaction, Patrick**
  **1965 Chandolin-près-Savièse (CH)**
• **Quièvre, Jacques**
  **77540 Rozay en Brie (FR)**
• **Morin, Jean-Benoît**
  **06510 Carros (FR)**

(74) Mandataire: **P&TS SA (AG, Ltd.)**
**Avenue J.-J. Rousseau 4**
**P.O. Box 2848**
**2001 Neuchâtel (CH)**

(56) Documents cités:
EP-A2- 1 253 404     WO-A2-2007/102134
GB-A- 2 415 788      US-A1- 2008 214 360

**Description**

<u>Domaine technique</u>

La présente invention concerne un procédé et un dispositif portable intégré muni d'un accéléromètre pour la mesure de paramètres biomécaniques de la foulée dans la course à pied.

<u>Etat de la technique</u>

Les résultats d'un coureur de course à pied dépendent essentiellement des qualités d'endurance cardiovasculaires et de l'efficacité mécanique de la foulée. On connaît dans l'état de la technique de nombreux dispositifs et tests pour mesurer les progrès en matière de performances cardiovasculaires. Par exemple, de nombreux coureurs s'entraînent avec un pulsomètre qui leur indique en tout temps leur rythme cardiaque, tandis que différents tests permettent par exemple de mesurer le volume maximal d'oxygène prélevé au niveau des poumons et utilisé par les muscles par unité de temps (V0$_2$max).

La présente invention concerne plus précisément les procédés et les dispositifs de mesure de l'efficacité biomécanique de la foulée. En améliorant l'efficacité de chaque foulée, le coureur parvient à courir plus vite ou plus longtemps avec une quantité d'énergie donnée. Il est cependant difficile, même pour un coureur accompagné par un entraîneur expérimenté, de mesurer objectivement l'efficacité de sa foulée et de l'améliorer sans recourir à des dispositifs de mesure. En particulier, il existe un besoin pour un procédé et un dispositif simple, portable, autonome, capable de fournir en temps réel et immédiatement après la course des paramètres utiles à l'analyse de la foulée.

Il est aussi difficile pour un coureur d'éviter le risque de surentraînement, de fatigue voire de blessure. Les signes avant-coureurs sont difficiles à percevoir et il n'existe pas dans l'art antérieur de dispositif simple permettant de détecter et d'évaluer le risque de fatigue musculaire ou de blessure.

On utilise fréquemment dans l'état de la technique des tapis de course munis de capteur de force par exemple qui permettent de mesurer avec une très grande précision la force de réaction du sol à chaque fois que le pied est en appui. Ces dispositifs sont cependant coûteux et réservés à un usage professionnel par des entraîneurs, des salles de gymnastique ou des hôpitaux par exemple. Ils permettent uniquement de mesurer la force lorsque le pied touche le sol, mais ne fournissent aucune indication quant au déplacement du coureur lorsque les pieds sont en l'air. Par ailleurs, le style de course sur un tapis diffère sensiblement de celui qui est adopté sur le terrain ou pendant une course de longue durée, en sorte que les indications fournies par ces appareils doivent être interprétées.

On connaît aussi des montres bracelets munies d'un capteur inertiel et destinées avant-tout aux coureurs occasionnels. Un exemple est décrit dans EP1862765. Les poignets d'un coureur suivent cependant une trajectoire bien différente de celle du centre de masse de l'athlète, et reviennent même brièvement en arrière lors de chaque pas. De tels dispositifs sont éventuellement utiles pour évaluer approximativement la distance parcourue ou le nombre de calories dépensées pendant une session de course, mais sont en revanche incapables d'analyser précisément la foulée.

Dans ce contexte, les expressions « centre de masse » et « centre de gravité » doivent être considérées comme équivalentes. Elles désignent le barycentre d'un coureur. Si, à cause de la position du coureur le centre de masse ou de gravité se situe au dehors du corps du coureur, le centre de masse ou de gravité coïncide avec le point le plus proche du centre de masse ou de gravité et appartenant au corps du coureur. En d'autres termes, le centre de masse est quasiment au de fixation du dispositif et/ou du capteur, sur le tronc, en général près de la taille.

US6397151 décrit un bracelet destiné aux sports de combats et muni d'un accéléromètre capable de mesurer une séquence d'accélérations de l'avant-bras durant un coup. DE4426302 décrit un autre système destiné à mesurer des accélérations lors de sports de combats. Ces dispositifs sont inadaptés à la course à pied.

Différentes sociétés proposent aussi de placer un accéléromètre dans ou sur la chaussure. Un exemple est décrit dans WO09094746. Cet emplacement permette de suivre de très près la trajectoire d'un des deux pieds. Il est cependant éloigné du centre de gravité du coureur et ne permet donc pas de détecter par exemple si le torse du coureur oscille ou balance de façon inadaptée. Par ailleurs, ce capteur ignore ce que fait l'autre pied, et ne permet pas de mesurer par exemple la durée de temps de vol du coureur à chaque foulée. Il ne permet pas davantage d'analyser le comportement du centre de gravité et donc ne permet pas une analyse précise de la foulée.

EP1897598 décrit un dispositif d'aide à l'entraînement pour différents sports. Il utilise un accéléromètre à la cheville ou aux genoux. A nouveau, ces différents emplacements sont éloignés du centre de gravité de l'athlète et de son plan de symétrie, en sorte que les mesures fournies sont imprécises ou non pertinentes pour l'analyse du style de course.

WO2009/094746 décrit un accéléromètre capable de déterminer lui-même son emplacement sur le pied, au poignet etc.

US6148280 décrit un système comportant des accéléromètres et des gyroscopes répartis sur tout le corps de l'athlète afin d'analyser la trajectoire du corps lors d'un mouvement. Les données fournies par les différents capteurs sont transmises à un ordinateur personnel à distance. Ce système donc ne permet pas de fournir en temps réel, immédiatement après une course, des paramètres utiles à l'analyse de la foulée. Ce système est particulièrement complexe, coûteux et fragile. Un dispositif comparable est aussi décrit dans EP418548.

On connaît par ailleurs des dispositifs munis d'un capteur inertiel permettant de mesurer des paramètres musculaires d'un athlète à l'aide de tests courts. US5474083 décrit par exemple un dispositif destiné à mesurer l'activité musculaire d'un athlète pendant des exercices courts de levers de poids. Ce dispositif est uniquement adapté à des tests courts, et pas à la course à pied par exemple. D'autres exemples sont aussi décrits dans les demandes de brevet WO07107491, EP2027817 ou WO2010046448 déposées par la demanderesse. Ces dispositifs sont avant tout destinés à la musculation et permettent de mesurer par exemple la force, la puissance ou la raideur des muscles à l'aide de séries de sauts par exemple. Bien que ces paramètres musculaires soient utiles au coureur également, il n'est pas suggéré de les utiliser pour l'analyse de la foulée, ni de les déterminer à partir de tests de course à pied.

WO2007036611, qui permet en outre de déterminer le poids maximal qu'une personne est capable de soulever, souffre des mêmes limitations.

US2009018794 décrit un dispositif pour mesurer la progression d'une personne en mouvement au moyen d'un capteur inertiel placé près du centre de gravité. La vitesse et les autres paramètres sont déterminés en fonction de l'accélération verticale maximale ou éventuellement de l'accélération verticale minimale en utilisant un facteur de proportionnalité k. Ce facteur k, qui n'est pas décrit dans le document, dépend cependant nécessairement du type de déplacement, du moyen de locomotion et du style de course ; les résultats calculés sont donc équivoques ou dépendent d'hypothèses invérifiables sur le facteur k. Le but de ce document est avant tout d'additionner des indications obtenues à chaque foulée afin d'afficher par exemple la distance parcourue, le nombre de pas ou la vitesse, mais pas d'afficher des paramètres permettant d'analyser l'efficacité de chaque foulée.

EP1754521 décrit un procédé permettant de déterminer l'efficacité de la foulée d'un coureur grâce à la mesure des accélérations instantanées du coureur. Les vitesses ne peuvent être déterminées qu'à une constante près. Ce dispositif calcule et affiche un indice d'efficacité global qui est le rapport de la somme des accélérations harmoniques sur les vitesses. L'utilisateur ne sait cependant ce qu'il doit faire pour améliorer cet indice global d'efficacité et le dispositif ne fournit pas d'autres paramètres liés à sa foulée et permettant au coureur d'améliorer directement son style de course.

WO2007017471 décrit un procédé et dispositif pour déterminer la vitesse d'un coureur au moyen d'un accéléromètre, en comptant le nombre et la fréquence de changements de sens de l'accélération verticale.

Les dispositifs comportant un capteur inertiel pour l'aide à la course déterminent en général la distance parcourue par une double intégration de l'accélération selon la direction horizontale. Cette mesure est cependant imprécise pour différentes raisons. Tout d'abord, le fait même d'intégrer produit une accumulation des erreurs et des imprécisions du capteur. La course produit des chocs importants à chaque foulée, en sorte que le signal d'accélération est très bruité, en particulier si l'accéléromètre est porté sur les membres, par exemple dans la chaussure, à la cheville ou au poignet. Par ailleurs, il est difficile de déterminer la direction antéropostérieure dans laquelle se fait la progression du coureur, et d'éliminer les composantes dues à l'accélération verticale ou transversale provoquée par le balancement du corps. Il en résulte une détermination de la distance parcourue imprécise, et un calcul de tous les paramètres basés sur cette distance qui souffre également de cette imprécision.

On connaît par ailleurs US5788655 qui décrit un appareil muni d'un accéléromètre et d'un affichage à cristaux liquides afin de mesurer le niveau d'activité de l'utilisateur et le nombre de calories consommées dans une journée. Ce dispositif n'est pas destiné à l'entraînement de sportifs et ne permet pas de mesurer les paramètres musculaires ou biomécaniques d'un athlète.

WO2005/074795 décrit un dispositif également destiné à être lié au corps d'une personne pour déterminer le niveau d'activité sur une longue période, par exemple un jour ou une semaine. Un dispositif comparable est également décrit dans WO03032826.

EP1992389 décrit un senseur muni d'un accéléromètre, d'un gyroscope, d'un récepteur GPS, d'un magnétomètre et d'un cardiomètre afin de suivre les efforts d'un sportif. Le dispositif est complexe et ne fournit pas d'indications utiles pour améliorer la foulée lors de la course à pied.

On connaît par ailleurs d'autres dispositifs basés sur des accéléromètres et des gyroscopes et qui permettent d'analyser la trajectoire d'un accessoire sportif, par exemple une batte de baseball (US5056783) ou un club de golf.

US20080288200 concerne un dispositif pour mesurer la posture et l'intensité d'une activité physique comprenant un accéléromètre triaxial. Ce dispositif permet de calculer des paramètres, notamment l'EEp (« Potential Energy Expediture ») et l'EEk (« Kinetic Energy Expediture ») qui ne dépendent pas de la distance parcourue par le coureur ni par le temps compté par un chronographe.

EP1253404 décrit un dispositif qui détermine l'évolution de la position d'un pédestre à partir de son contexte et qui permet de calculer la distance totale parcourue par l'utilisateur, sa vitesse ainsi que la distance entre deux impacts au sol.

US5976083 concerne un dispositif pour surveiller, estimer et prévoir le niveau de fitness d'un utilisateur. Le dispositif comprend un système de fixation autour de la poitrine de l'utilisateur, à niveau de son coeur. Le dispositif utilise des données personnelles de l'utilisateur, les accélérations verticales données par un accéléromètre et l'horloge en temps-réel pour calculer la distance parcourue par l'utilisateur. Les paramètres calculés lors de l'estimation/prévision de fitness du dispositif comprennent la durée de la session, le total des calories brulées, la distance parcourue, le fitness, la moyenne du rythme cardiaque, la pente et l'intercepte de l'EE (Energy Expenditure) par rapport au rythme cardiaque. Ces résultats sont donnés sur demande de l'utilisateur.

WO2006030065 décrit un dispositif pour la reconnaissance du mouvement d'un être humain par un capteur de mouvement porté sur le tronc, sur les chaussures ou sur le poignet d'un être humain. Un signal de mesure est comparé à un signal de référence prédéterminé qui peut être mesuré de manière similaire chez un être humain connu. Une opération de reconnaissance est effectuée en fonction de la comparaison entre le signal de mesure et le signal de référence prédéterminé, l'opération de reconnaissance aboutissant à la reconnaissance ou la non-reconnaissance. Ce dispositif n'est adapté pour analyser des paramètres biomécaniques de la foulée d'un coureur, le but étant la reconnaissance du mouvement d'un être humain, pour des identifications et/ou vérifications.

EP1764583 concerne un dispositif pour mesurer des informations d'un coureur telles que le temps total de course, la vitesse moyenne et maximale, la distance totale parcourue et le rythme cardiaque moyen et maximal. Le dispositif, qui comporte un accéléromètre mesurant seulement l'accélération verticale, est fixé sur le tronc du coureur et communique avec un autre dispositif-récepteur porté sur le poignet du coureur, sur lequel les informations sont affichées.

WO2005091113 décrit un dispositif et procédé d'évaluation du mouvement d'un piéton, consistant à déterminer au moins une position d'au moins une partie identifiée du piéton, à projeter la ou les positions sur au moins un plan et à calculer le mouvement à partir de la positon ou des positions projetées sur ce plan. Le dispositif, dépourvu de chronographe, permet de déterminer seulement et de façon compliquée l'orientation des membres et l'orientation et la longueur d'un pas.

Tous ces documents ne permettent pas de déterminer de façon simple et immédiate des paramètres biomécaniques de la foulée d'un coureur liés à son centre de gravité, notamment liés au déplacement de son centre de gravité. L'expression « déplacement vertical du centre de masse ou de gravité » désigne son abaissement et/ou son élévation par rapport à son déplacement idéal, qui se déroule dans un plan horizontal. En d'autres termes cette expression désigne l'ondulation du centre de masse ou de gravité par rapport au plan horizontal auquel le centre de masse ou de gravité appartenait au début de la course, cette ondulation résultant de la somme de l'abaissement et de l'élévation du centre de masse ou de gravité par rapport à ce plan horizontal.

Ce paramètre est très important pour un coureur qui parcourt des brèves distances, par exemple 200 m, dans un bref délai de temps : afin de courir de façon la plus efficace possible, son centre de gravité idéalement ne doit pas se déplacer verticalement. Ce paramètre est aussi important pour un marathonien, qui au contraire exploite l'oscillation verticale du centre de masse et donc le rebondissement élastique de ses jambes au sol.

Il existe donc un besoin pour un dispositif et procédé qui permette de déterminer de façon simple et immédiate des paramètres biomécaniques de la foulée d'un coureur liés à son centre de gravité, notamment liés au déplacement vertical de son centre de gravité.

US20080190202 concerne un appareil qui est en mesure d'identifier la position de son attache sur la base de mesures d'accélération, et de sélectionner un algorithme d'analyse du mouvement dans une base de données sur la base de cette position d'attache. L'appareil permet de calculer différents paramètres musculaires.

WO2007102134 concerne la rééducation de patients présentant des invalidités motrices. Il décrit comment évaluer l'utilisation fonctionnelle d'un membre en déterminant le synchronisme des mouvements du patient selon la cyclicité du mouvement.

GB2415788 concerne le calcul d'un indice tel que l'indice de fatigue, à partir des valeurs mesurées par des signaux électromyographies (EMG) et d'autres paramètres liés à l'exercice. Les autres paramètres comprennent les mouvements du corps mesurés par des capteurs d'inertie tels que des accéléromètres. US2008/0214360 A1 divulgue une méthode et un dispositif pour estimer les paramètres biomécaniques d'un coureur.

Bref résumé de l'invention

Un but de la présente invention est de proposer un procédé et un dispositif exempt des limitations des procédés et dispositifs connus, en particulier un procédé et un dispositif qui permette de déterminer de façon simple et immédiate des paramètres biomécaniques de la foulée d'un coureur liés à son centre de gravité, notamment liés au déplacement vertical de son centre de gravité.

Un autre but de l'invention est de proposer un dispositif et un procédé plus précis, plus économique, plus compact, plus facile à manipuler et/ou qui fournisse des résultats pratiquement instantanément et en temps réel.

La présente description divulgue un dispositif et un procédé pour mesurer et analyser la cinématique du mouvement (accélération, vitesse et position du centre de masse d'un coureur) ainsi que de manière beaucoup plus large l'ensemble des paramètres qui caractérisent les qualités techniques et les capacités physiques du coureur.

La présente description divulgue aussi un dispositif et un procédé autonome et portable, permettant de déterminer différents paramètres utiles au coureur à pied afin d'améliorer la régularité et l'efficacité de sa foulée, et de réduire le risque de fatigue ou de blessure.

L'invention part notamment de la constatation que tout un ensemble de paramètres biomécaniques peuvent, parmi lesquels l'abaissement et l'élévation du centre de masse, de façon inattendue, être mesurés et calculés avec une précision significative au moyen d'un simple accéléromètre judicieusement placé. Certains de ces paramètres n'avaient jamais pu être mesurés auparavant, tandis que d'autres l'étaient seulement avec des dispositifs beaucoup plus complexes et non-autonomes, par exemple en exigeant une analyse séparée sur un ordinateur portable pour extraire certains paramètres.

Selon l'invention, ces buts sont atteints notamment au moyen d'un dispositif et d'un procédé selon les revendications 1 et 11.

Ce procédé offre notamment l'avantage de permettre une mesure et un calcul de paramètres fiables, même avec un appareil simplifié. La fiabilité est notamment obtenue par une fixation de l'accéléromètre près du centre de gravité du coureur, au moyen d'une ceinture ou d'un clip. La durée de la course est déterminée par le chronographe intégré au dispositif. La mesure de la distance totale parcourue ne dépend pas non plus de la précision de l'accéléromètre, soit parce qu'elle est implicite et prédéterminée, soit par l'utilisateur qui la choisit/l'introduit, ou encore par récepteur satellitaire de type GPS par exemple.

L'invention part aussi de la constatation surprenante que plusieurs paramètres utiles à l'entrainement de la course à pied peuvent être déterminés sur la base de mesures d'accélération effectuées avec le dispositif décrit ci-après. L'invention concerne ainsi différents moyens et étapes de procédé permettant de calculer un paramètre comprenant un indice de régularité de la foulée d'une façon différente de l'art antérieur, en utilisant exclusivement les mesures d'accélération, et avec une précision remarquable. La présente description divulgue un dispositif capable de calculer et d'afficher toute une série de paramètres biomécaniques directement utilisables par le coureur à pied ou son entraîneur.

La présente description offre ainsi un nouveau dispositif et un nouveau procédé pour calculer des paramètres biomécaniques de la foulée qui étaient soit calculés différemment dans l'art antérieur, avec des moyens plus complexes (gyroscopes triaxiaux, ordinateur portable, etc....), ou mêmes des paramètres qu'il était impossible de calculer ou que personne n'avait pris la peine de mesurer.

La position du dispositif dans le creux du dos ou sur le ventre permet de déterminer toute asymétrie entre les mouvements effectués par chacune des deux jambes à chaque foulée. Il est ainsi possible de mesurer l'asymétrie des paramètres musculaires et biomécaniques des deux jambes avec un seul capteur judicieusement placé et un traitement adéquat des signaux.

Les paramètres biomécaniques calculés peuvent inclure par exemple la distance parcourue par le centre de masse du coureur pendant la durée où le pied est en contact avec le sol (distance sur l'appui). Le début et la fin de la durée est déterminée sur la base de la mesure d'accélération, par exemple de l'accélération selon la direction verticale ou antéropostérieure, qui change brusquement lorsque le pied quitte ou atteint le sol. La mesure de cette distance est obtenue par la multiplication de la vitesse selon la direction antéropostérieure avec le temps de contact. Comme l'accéléromètre est placé près du centre de masse du coureur, il est possible aussi de déterminer avec précision la vitesse verticale de décollage ainsi que l'angle d'envol du centre de gravité.

Les paramètres biomécaniques calculés peuvent aussi inclure par exemple l'angle de pose de la jambe sur le sol ainsi que l'angle du secteur d'appui de la jambe, qui est l'angle formé par la jambe entre le moment où le pied touche le sol et le moment où le pied quitte le sol. Dans l'art antérieur, ces différents angles étaient déterminés sur la base d'images vidéo haute fréquence, et nécessitaient donc un équipement complexe incapable de fournir un résultat en temps réel. Il a été découvert dans le cadre de l'invention que des opérations de traitement de signal et de calcul à partir de séquences de données d'accélération fournissent un moyen plus simple, plus économique et fiable de mesurer ces angles de manière pratiquement instantanée.

Le procédé et dispositif permet aussi de déterminer de manière nouvelle et avec des nouveaux moyens un indice de régularité de la foulée. L'indice de régularité dépend par exemple de la dispersion des différents paramètres mesurés à chaque foulée, par exemple de la dispersion de la réactivité, ou de la dispersion d'un autre paramètre lié à la foulée.

De manière générale, le procédé permet aussi de déterminer de manière nouvelle et avec de nouveaux moyens un indice d'efficacité de la foulée sur la base de la distance d'appui de la jambe, du temps de contact au sol, et de la raideur de la jambe du coureur, de la longueur de la foulée.

De manière générale, le procédé permet d'indiquer au moins un indicateur de niveau de fatigue musculaire, c'est-à-dire un indicateur dont la valeur absolue ou l'évolution temporelle peut être utilisée par exemple pour révéler une fatigue anormale. L'utilisation d'un accéléromètre pour déterminer le niveau de fatigue offre des possibilités très simples pour un athlète d'optimiser son entraînement en évitant le risque de plafonnement des performances en cas de surentraînement.

Les paramètres biomécaniques calculés peuvent aussi inclure par exemple une asymétrie entre au moins un paramètre de la jambe gauche et au moins un paramètre correspondant de la jambe droite. Par exemple, le dispositif peut détecter et afficher un paramètre permettant de déduire que la longueur des foulées parcourues avec une jambe en appui est plus longue qu'avec l'autre, ou des angles de pose ou d'envol différents. Ces différences peuvent par exemple correspondre à des différences de longueur de jambe ou d'entraînement musculaire (notamment lorsque ces différences subsistent pendant des longues périodes ou lors de plusieurs entraînements successifs) ; elles peuvent aussi être des signes indicateurs ou annonciateurs de blessure, de fatigue. La description concerne donc aussi un usage nouveau d'un accéléromètre comme dispositif de détection de risque de blessure lors de la course à pied.

En outre, le procédé permet d'indiquer au moins un indicateur de risque de blessure, c'est-à-dire un indicateur dont la valeur absolue ou l'évolution temporelle révèle un risque de blessure. Par exemple, une variation rapide, ou à des niveaux inhabituels, de la raideur des muscles d'une jambe est souvent annonciatrice d'une blessure ; cette variation peut être détectée et affichée pour protéger le coureur.

Les paramètres biomécaniques calculés peuvent aussi inclure par exemple la vitesse verticale du centre de masse au cours d'une foulée, et/ou le déplacement vertical du centre de masse au cours d'une foulée et/ou l'angle d'envol du centre de masse. Un style de course efficace implique généralement un déplacement du centre de masse du coureur selon une direction aussi horizontale que possible, avec une vitesse verticale aussi constante et proche de zéro que possible, et une vitesse horizontale dans l'axe antéropostérieure aussi constante et élevée que possible. Une séquence de données d'accélération selon au moins la direction antéropostérieure est de préférence également mesurée par ledit accéléromètre et traitée séparément de la séquence de données d'accélération selon la direction verticale afin de calculer au moins certains des paramètres.

Les paramètres biomécaniques calculés peuvent aussi inclure par exemple la cadence des pas, le temps de contact avec le sol, le temps de vol, l'accélération moyenne sur appui, la force maximale sur appui, la vitesse horizontale du centre de masse sur appui, la raideur ou la réactivité des muscles, par exemple des muscles d'une jambe ou des deux jambes de manière différenciée.

Dans un mode de réalisation, une séquence des données d'accélération selon au moins la direction latérale est également mesurée et traitée séparément de la séquence des données d'accélération selon la direction verticale afin de calculer au moins certains des paramètres, par exemple la rotation et le balancement du tronc à chaque foulée. Ce capteur peut aussi être utilisé pour distinguer entre les foulées effectuées avec le pied gauche et celles qui sont effectuées avec le pied droit.

La direction verticale est déterminée par exemple par mesure de la gravité lorsque le coureur est à l'arrêt. La direction antéropostérieure est déterminée ou corrigée avantageusement lors de la course en déterminant la direction spatiale dans lequel le déplacement horizontal est le plus important.

Une transformation de référentiel « dispositif - coureur » est de préférence effectué étant donné que les axes de l'accéléromètre ne coïncident jamais parfaitement avec les axes verticaux respectivement horizontaux et latéraux du coureur. Par conséquent, les données triaxiales de l'accéléromètre sont projetées le long de l'axe vertical, horizontal et/ou respectivement latéral du coureur. Ainsi, le dispositif fournit une, deux ou trois séquences de données d'accélération qui ne correspondent pas nécessairement aux axes de l'accéléromètre et qui sont traitées et utilisées indépendamment pour le calcul des paramètres.

Le dispositif propose avantageusement un plan d'entraînement personnalisé en fonction des paramètres et destiné à améliorer les qualités techniques ou musculaires du coureur. Par exemple, le plan d'entraînement peut être optimisé pour entraîner de préférence les points faibles de l'athlète. Les exercices peuvent comporter des exercices différents de la course, y compris des sauts, des exercices musculaires, d'étirement etc. Le dispositif peut aussi être utilisé lors de ces exercices pour surveiller l'exécution dudit plan d'entraînement.

Le dispositif peut aussi distinguer entre des foulées effectuées sur des segments de parcours au plat, à la montée ou en descente, et affiche des paramètres et/ou des recommandations différenciées pour ces trois types de segments. La détermination de la pente peut être effectuée par l'accéléromètre, avec un récepteur satellitaire, un capteur de pression ou sur la base d'indications fournies par le coureur.

Le système permet, en fonction de la typologie du coureur, de conseiller le type de chaussure idéale pour améliorer l'efficacité du déplacement et la protection du système musculotandineux.

Brève description des figures

Des exemples de mise en oeuvre de l'invention sont indiqués dans la description illustrée par les figures annexées dans lesquelles :

La figure 1 illustre un exemple de dispositif.
La figure 2 est un schéma-bloc simplifié des composants électroniques du dispositif.
La figure 3 illustre de manière schématique le placement du dispositif sur le tronc du coureur au moyen d'une ceinture (sur le creux du dos ou sur le ventre afin d'être le plus proche possible du centre de gravité).

La figure 4 illustre un exemple de mesure de l'accélération selon la direction verticale et antéropostérieure au cours d'une foulée.

La figure 5 illustre schématiquement les oscillations du centre de masse du coureur au cours d'une foulée (ondulation, élévation, abaissement, angle d'envol du centre de gravité).

La figure 6 illustre schématiquement l'angle de pose de la jambe, l'angle de secteur d'appui de la jambe et la distance sur appui.

Exemple(s) de mode de réalisation de l'invention

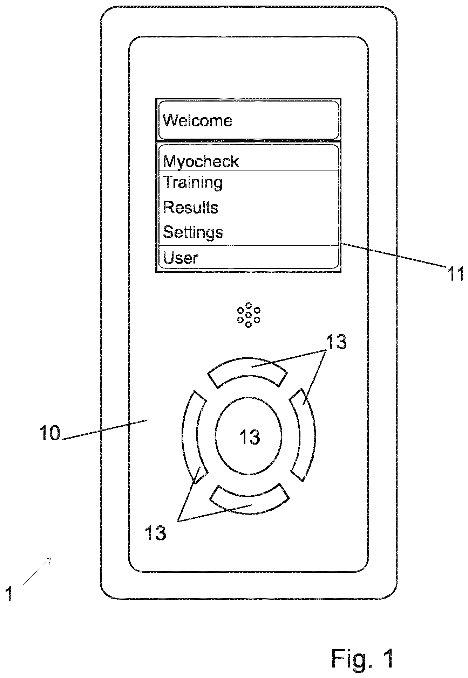

Un exemple de dispositif est illustré sur la figure 1. Ce dispositif 1 comporte un boîtier, par exemple un boîtier en plastique avec un poids inférieur à 100 grammes, de préférence moins de 50 grammes (y compris le contenu). L'athlète peut attacher ce boîtier à sa hanche, dans le creux du dos, ou sur le ventre au moyen d'une ceinture, par exemple une ceinture extensible fermée par un ruban Velcro, ou d'un clic directement fixé sur les habits. La ceinture permet une fixation stable près du centre de gravité du coureur, en minimisant les vibrations et déplacements du dispositif par rapport au centre de masse.

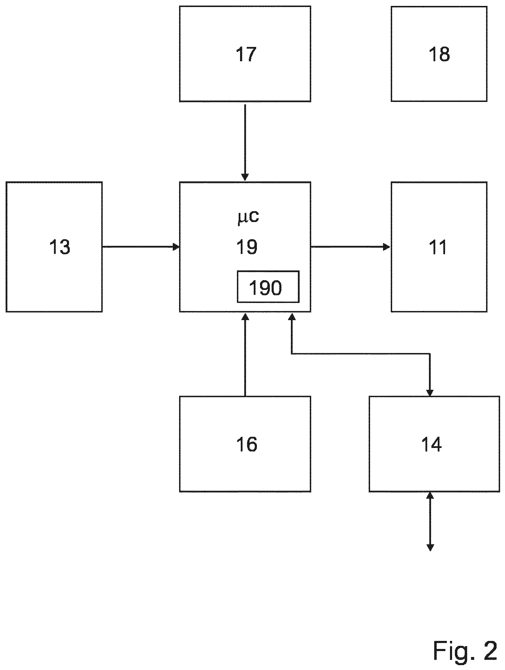

Le dispositif comporte en outre un affichage 11, par exemple un affichage alphanumérique ou graphique, de préférence un affichage à cristaux liquides. Cet affichage permet d'afficher des menus de contrôle, l'état de la mémoire, l'état de la batterie 18 (figure 2), ainsi que des paramètres numériques et qualitatifs déterminées pendant et après les tests. Des organes de contrôle 13, par exemple des boutons ou d'autres organes tactiles, permettent de naviguer dans les menus, de sélectionner des options, d'entrer des données et de choisir les résultats à afficher. Dans l'exemple illustré, le dispositif comporte quatre boutons de navigation arrangés autour d'un bouton de confirmation central.

Le dispositif comporte de préférence un accéléromètre triaxial 17, par exemple un accéléromètre basé sur un composant de type MEMS, et fournissant des séquences d'accélérations distinctes selon ces trois axes. L'orientation du dispositif et/ou de l'accéléromètre relativement au coureur est mesurée à l'arrêt, et permet ainsi la calibration et la transformation du référentiel « dispositif-coureur » afin de mesurer des accélérations dans un référentiel lié au coureur. Dans une variante, les données triaxiales de l'accéléromètre sont projetées et mémorisées selon un seul des axes, ou selon deux axes ou selon trois axes. L'accéléromètre peut avoir un axe privilégié offrant une précision, une résolution, une gamme de mesure et/ou une fréquence d'acquisition plus élevée que selon les autres axes. Cet axe privilégié est avantageusement orienté sensiblement verticalement lors d'un usage normal, afin d'améliorer la qualité de la mesure selon la direction verticale. La gamme de mesure selon cet axe privilégié est de préférence supérieure à $\pm$8G, ou même supérieure à $\pm$10G. La résolution de cet axe est de préférence supérieure à 10 ou même 12 bits.

Afin de réduire son coût, sa consommation, son encombrement, et sa complexité, le dispositif est avantageusement dépourvu de gyroscope. L'usage d'un gyroscope à un ou plusieurs axes peut cependant être envisagé pour certaines applications ou pour calibrer et orienter de façon plus fiable le dispositif.

La figure 2 est un schéma bloc simplifié des principaux composants du système. Le dispositif 1 est de préférence autonome électriquement et inclut une batterie 18, par exemple un accumulateur rechargé via une prise USB ou un chargeur approprié. La batterie alimente en particulier un microprocesseur 19 ou un microcontrôleur muni d'une mémoire RAM et/ou EEPROM 190. Le microprocesseur exécute un programme en mémoire EEPROM, qui peut être mis à jour via une interface USB ou Bluetooth par exemple, afin de contrôler l'affichage 11 et d'analyser les données d'accélération fournies par l'accéléromètre. Ce programme permet donc de contrôler les moyens de calcul afin de déterminer des paramètres biomécaniques de la foulée sur la base des séquences de données d'accélération mesurées selon un ou plusieurs axes.

Le dispositif inclut également un chronographe pour mesurer des durées temporelles $\Delta t$, par exemple un chronographe électronique basé sur une horloge en temps-réel RTC 16, ainsi qu'un haut-parleur ou un buzzer non représenté commandé par le microprocesseur 19 pour générer des alarmes ou des sons. Un module d'entrée-sortie UART 14 permet d'échanger des données avec des dispositifs externes, par exemple pour le reprogrammer et/ou pour transmettre des résultats de mesure à un ordinateur personnel, un téléphone mobile ou tout autre dispositif de traitement de données à proximité. Le module UART peut par exemple être connecté à un connecteur filaire de type USB, ou à une interface sans fil de type Bluetooth ou Zigbee par exemple. Il est aussi possible de prévoir un connecteur pour carte mémoire Flash permettant de stocker des résultats ou de charger un nouveau programme ou un plan d'entraînement par exemple.

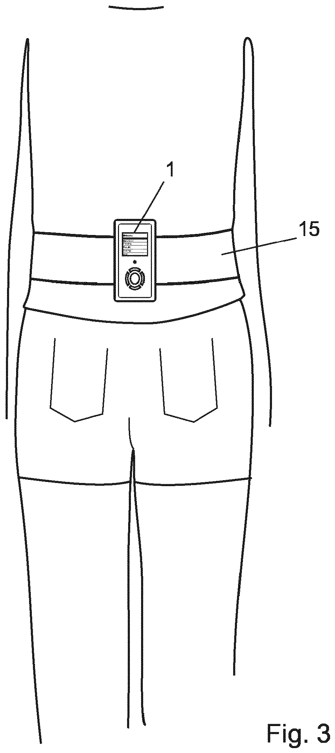

La figure 3 illustre de manière schématique le placement du dispositif 1 dans le creux des reins (creux du dos) d'un utilisateur 2, au moyen d'une ceinture 15. Ce placement permet d'assurer une position très proche du centre de masse du coureur pendant pratiquement toute la foulée, sans que le dispositif puisse vibrer ou se déplacer par rapport au centre de masse. Le dispositif peut aussi être placé de manière toute aussi avantageuse sur le ventre. Des essais ont montré qu'un positionnement du dispositif sur les membres (par exemple au poignet, sur le pied ou près du genou), ne permet absolument pas de faire ce genre de mesure et d'analyse, et exclut donc toutes les applications et inventions de l'art antérieure qui décrivent des dispositifs placés ailleurs que près du centre de gravité du sportif.

Au terme et/ou lors d'une course à pied, le dispositif mesure ou détermine par traitement du signal les quatre paramètres de base suivants :

Acc (séquence de données d'accélération)
$T_c$ (temps de contact)
$T_v$ (temps de vol)
T (temps de course).

Par ailleurs, les paramètres suivants sont de préférence introduits par l'utilisateur avant ou après la course :

D (distance de course)
H (taille en centimètres de l'utilisateur)
M (masse utilisateur).

L'accélération Acc est de préférence obtenue selon plusieurs axes distincts.

Par rapport à d'autres dispositifs de l'art antérieur, le dispositif fournit donc plusieurs séquences d'accélération distinctes, analysées et stockées indépendamment les unes des autres. Dans un mode de réalisation préférentiel, le dispositif détermine une séquence de données d'accélération selon la direction verticale. Dans un mode de réalisation préférentiel, le dispositif détermine en outre l'accélération selon la direction antéropostérieure dans laquelle le coureur avance. Dans un mode de réalisation préférentiel, le dispositif peut aussi fournir une troisième séquence de données d'accélération selon la direction latérale, afin par exemple de déterminer le balancement latéral du corps de l'athlète. Ces trois directions peuvent correspondre à des axes éventuellement différents des axes de l'accéléromètre.

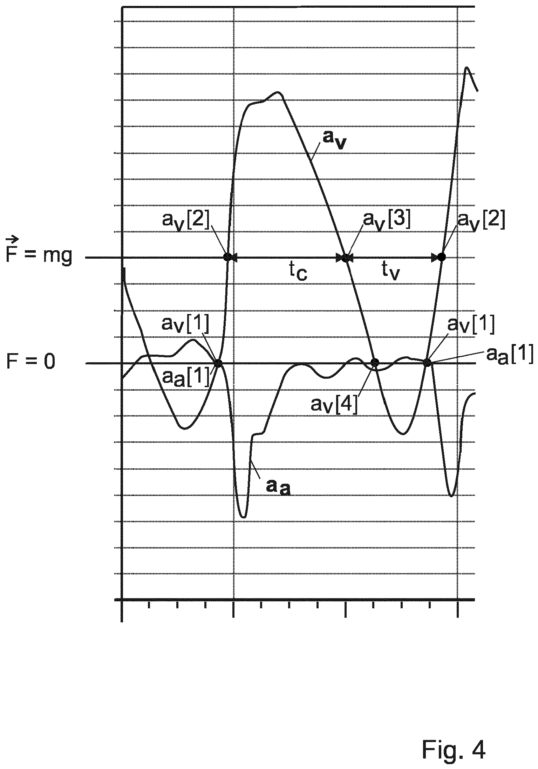

La figure 4 illustre deux courbes montrant les valeurs d'accélération $a_v$ selon la direction verticale et $a_a$ selon la direction antéropostérieure au cours d'une foulée. La direction antéropostérieure correspond à la direction horizontale dans laquelle le coureur progresse, c'est-à-dire une direction horizontale perpendiculaire au rachis. L'orientation du dispositif et/ou de l'accéléromètre relativement au coureur est mesurée à l'arrêt, et permet ainsi la calibration et la transformation du référentiel « dispositif-coureur ». La direction antéropostérieure peut être approximée initialement, puis corrigée après un tronçon de course rectiligne en mesurant la direction dans laquelle le coureur parcourt la plus grande distance. Avantageusement, le référentiel dans lequel les séquences de données d'accélération sont mesurées est lié au coureur, et tourne donc lorsque le coureur tourne autour d'un stade par exemple. Une correction de référentiel est de préférence effectuée après la calibration, et/ou en cours d'exercice, si les axes de l'accéléromètre du dispositif ne coïncident pas avec les axes verticaux et antéropostérieurs, par exemple en cas de placement incorrect ou imprécis du dispositif.

A l'instant $a_v[1]$, le coureur pose le talon sur le sol. L'accélération verticale vers le haut exercée par le sol sur le pied augmente rapidement, tandis que l'accélération antéropostérieure aa devient négative - le coureur est freiné par le choc du talon sur le sol.

L'accélération verticale augmente ensuite rapidement jusqu'à ce que le pied soit complètement en appui sur le sol à l'instant $a_v[2]$. A ce moment le centre de gravité pivote vers l'avant autour de la jambe en appui.

Entre l'instant $a_v[3]$ et l'instant $a_v[4]$, le pied du coureur commence à redécoller; l'accélération verticale diminue rapidement à cet instant, tandis que l'accélération antéropostérieure est proche de son maximum et que le coureur est propulsé vers l'avant.

A partir de l'instant $a_v[4]$ et jusqu'au prochain instant $a_v[1]$, le coureur est en l'air; l'accélération verticale est alors négative et correspond à la gravité. Le centre de gravité du coureur parcourt une trajectoire balistique avec une vitesse horizontale sensiblement constante et une trajectoire sensiblement parabolique.

Le temps de contact durant la foulée correspond donc à l'intervalle $t_c$ entre les instants $a_v[2]$ où le pied est en contact avec le sol, et $a_v[3]$ où le même pied redécolle. Le temps de vol $t_v$ correspond à la période complémentaire entre $a_v[3]$ et $a_v[2]$. Ces deux durées peuvent donc être déterminées directement à partir des séquences de données d'accélération $a_v$ et éventuellement $a_a$, par simple traitement de signal et détermination de points particuliers durant la foulée. Dans un mode de réalisation préférentiel, le temps de vol et le temps de contact sont calculés en effectuant la moyenne des temps de vol et des temps de contact au cours de l'ensemble des foulées effectuées lors d'un test ; les foulées les moins représentatives, par exemple au début du test, à la fin du test, lorsque la valeur s'éloigne trop de la moyenne, ou lorsque l'accélération antéropostérieure ou latérale prend des valeurs inhabituelles, sont de préférence écartées du calcul de la moyenne.

La mesure des paramètres biomécaniques de la foulée lors de course à pied est avantageusement effectuée pendant et immédiatement à la suite d'un test de course sur un circuit de course étalonné, par exemple en stade, sur une route, en salle de gymnastique, ou en utilisant un récepteur satellitaire de type GPS afin de s'affranchir des imprécisions liées à la mesure de distance parcourue avec un accéléromètre, et des chocs perturbant l'accéléromètre en cas de course sur un terrain accidenté.

La durée de l'exercice est de préférence déterminée à l'aide du chronographe intégré. Dans un mode de réalisation, le dispositif invite le coureur à courir à une vitesse prédéterminée, soit de façon qualitative (« cours lentement », « cours 400 mètres à ton rythme » etc.), soit de façon quantitative en émettant par exemple un signal sonore pour inviter l'utilisateur à ralentir ou au contraire à accélérer.

Le début de l'exercice est par exemple donné par l'appareil qui peut afficher ou restituer un compte à rebours, par l'utilisateur qui presse un bouton, ou détecté par l'accéléromètre dès que l'utilisateur se met à courir après le démarrage du programme. De la même façon, la fin de l'exercice peut être indiquée par l'utilisateur, ou détectée par l'accéléromètre dès que l'utilisateur a parcouru la distance attendue ou dès qu'il décélère suffisamment.

La distance parcourue est de préférence introduite par l'utilisateur dans le dispositif. Par exemple, le dispositif peut afficher un message invitant l'utilisateur à parcourir une distance prédéterminée, par exemple 100 mètres, 400 mètres, 1000 mètres ou davantage, sur piste ; les paramètres biomécaniques de la foulée sont ensuite affichés au terme de cet exercice. L'utilisateur peut aussi introduire lui-même la distance totale parcourue, ou la choisir dans un menu. Dans une autre variante il est possible d'analyser dans le temps des séquences successives et d'en extraire les variations des différents paramètres décrits plus haut.

Dans une variante, la distance parcourue est déterminée par un récepteur satellitaire, par exemple un récepteur GPS, intégré ou connecté au dispositif. Il est éventuellement envisageable, mais moins précis, de déterminer cette distance par une double intégration de l'accélération selon la direction antéropostérieure.

Les autres données qui sont de préférence introduites manuellement par l'utilisateur incluent sa masse en kilos et sa taille en centimètres. Des tests de plausibilité peuvent être effectués lors du test, sur la base des résultats de mesure de l'accéléromètre ; par exemple, la longueur de foulée doit être dans une relation plausible avec la taille de l'utilisateur.

Au terme (ou au cours) de l'exercice, le dispositif calcule un certain nombre de paramètres biomécaniques nouveaux, relatifs à la foulée du coureur, sur la base des paramètres ci-dessus mesurés par le dispositif et/ou introduits par l'utilisateur.

La vitesse moyenne $V_{moy}$ sur la distance D parcourue dans un temps de course T est calculée et affichée sur la base de la relation

$$V_{moy} = D/T$$

Des tests ont montré que l'utilisation de la vitesse moyenne $V_{moy}$ calculée sur la base de la distance et du temps total s'avère, de manière surprenante, plus précise et plus fiable que l'utilisation de la vitesse instantanée V lors de chaque foulée qui pourrait être obtenue par intégration de l'accélération antéropostérieure. Il est cependant envisageable, dans un mode de réalisation, d'utiliser cette vitesse instantanée mesurée. Afin de simplifier la notation, les formules suivantes utilisent la vitesse instantanée V ; il est cependant nécessaire de comprendre qu'il est préférable, dans la plupart des équations, d'utiliser la vitesse moyenne $V_{moy}$ calculée sur la base de la distance et du temps de parcours, ou éventuellement la vitesse donnée par un éventuel GPS.

Le nombre N de foulées par minutes pendant la période considérée est l'inverse de la durée moyenne d'une foulée $T_v + T_c$ pendant cette période:

$$N = 1 / (T_v + T_c)$$

La longueur moyenne de la foulée $L_f$ peut être calculée sur la base de vitesse moyenne sur le parcours. La longueur de foulée vaut alors

$$L_f = V*(T_c+T_v).$$

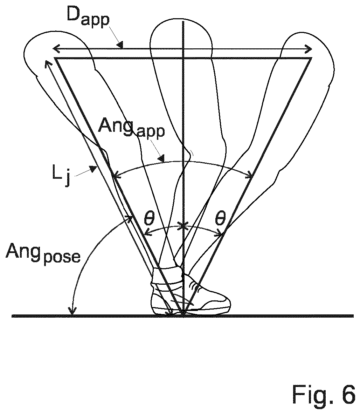

Un autre paramètre utile pour l'évaluation de l'efficacité de la foulée est constitué par la distance $D_{appui}$ parcourue par le centre de gravité CG pendant que le pied est en appui sur le sol. Il a été constaté que ce paramètre peut également être obtenu à partir des données d'accélération, par exemple avec la relation

$$D_{appui} = V*T_c$$

ou en utilisant la vitesse instantanée lors de chaque foulée ou de chaque appui. La figure 6 illustre de façon schématique et approximative la distance $D_{appui}$.

La vitesse $V_{deco}$ selon la composante verticale au moment du décollage du pied est fonction de l'accélération moyenne $A_{moy}$ et du temps de contact $T_c$ :

$$V_{deco} = A_{moy} * T_c / 2$$

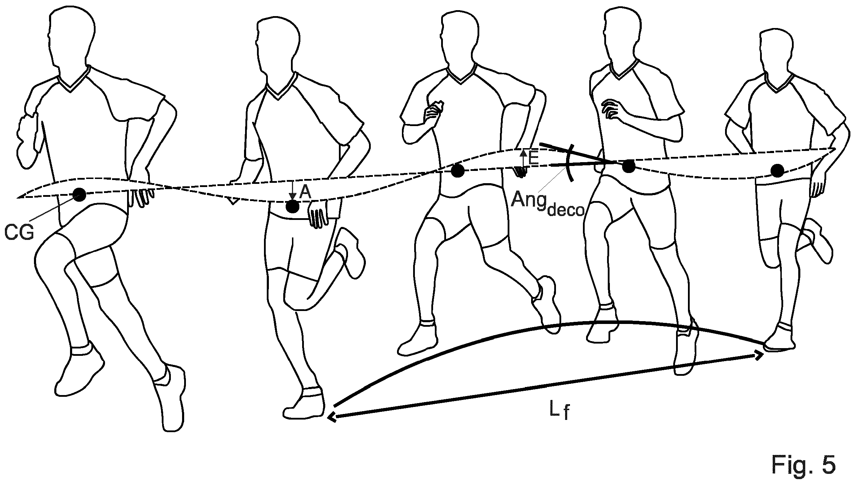

Il a aussi été constaté que l'angle de décollage du centre de gravité $Ang_{deco}$, illustré sur la figure 5, fournit des informations très utiles pour évaluer et améliorer la foulée. Il a en outre été constaté pour la première fois qu'une caméra n'est pas indispensable pour mesurer cet angle, qui peut être évalué de manière fiable à partir des séquences d'accélérations fournies par un accéléromètre tel que décrit, en particulier les séquences d'accélération dans la direction verticale. L'angle de décollage indique l'angle formé par la trajectoire du centre de gravité et la direction antéropostérieure. Cet angle de décollage peut être obtenu à partir de la vitesse moyenne $V_{moy}$ et de la vitesse verticale $V_{deco}$ au moment du décollage grâce à la relation

$$Ang_{deco} = 180/\pi * \tan{-1}(V_{moy}/V_{deco})$$

De la même façon, il est possible de déterminer sans caméra et à partir des données d'accélération l'angle de secteur d'appui $Ang_{app}$ (illustré sur la figure 6 et correspondant à l'angle parcouru par la jambe de longueur $L_j$ lorsque le pied est en appui) avec la formule :

$$Ang_{app} = 180 / \pi * (V_{moy}*T_c / L_j)$$

L'angle de pose de la jambe au moment où le pied rejoint le sol, est également illustré sur la figure 6. Il peut être estimé, toujours à partir des données de l'accéléromètre et pour la première fois sans caméra, à l'aide de la relation :

$$Ang_{pose} = (180 - Ang_{app}) / 2$$

La force maximale $F_{max}$ exercée par le sol sur le pied d'un coureur de masse M peut être déterminée à l'aide de la relation :

$$F_{max} = M*g*\pi/2 * (T_v / T_c+1)$$

La force moyenne sur appui normalisée par rapport au poids de corps $F_{moy}$ vaut quant à elle

$$F_{moy} = A_{moy} / g+1$$

L'abaissement A du centre de masse à chaque foulée peut être obtenu comme une fonction de la force d'appui maximale $F_{max}$, du temps de contact $T_c$ et de la masse du coureur M avec la formule

$$A = f(F_{max}, T_c, M) = |-(F_{max}*T_c^2) / (M*\pi^2) + (G*T_c^2)/8|$$

De la même façon, l'élévation E du centre de masse peut être calculée avec la formule

$$E = V_{deco}^2/(2*G)$$

dans lequel $V_{deco}$ correspond à la vitesse verticale au décollage.
La figure 5 illustre schématiquement la signification des paramètres A et E au cours d'une foulée. Un style de course efficace implique généralement un abaissement et une élévation réduits du centre de gravité CG qui se déplace idéalement dans un plan horizontal. Il est possible de calculer un paramètre d'ondulation du centre de gravité :

$$Ondulation = Abaissement + Elévation$$

qui résulte de la somme de ces deux paramètres.

Beaucoup de coureurs s'intéressent aussi à la raideur musculaire, qui renseigne sur la capacité des muscles des jambes à résister à la déformation lors de chaque impact au sol ou si au contraire ils ne produisent plus une tension suffisante nécessaire pour éviter un abaissement trop important du centre de gravité qui nuit à la performance. Un paramètre de raideur peut être calculé en fonction de la force maximale $F_{max}$ exercée par le sol sur le pied et de l'abaissement A, dont la valeur correspond généralement à l'instant où cette force maximale est exercée :

$$Raideur = F_{max}/A$$

Beaucoup de coureurs s'intéressent aussi à la régularité de leur foulée ; il est plus efficace de courir de façon régulière que de façon irrégulière. Les dispositifs de l'art antérieur ne permettent cependant pas de mesurer aisément la régularité de la foulée. Il a été constaté cependant que les séquences de données d'accélération peuvent être utilisées pour calculer un indice de régularité fiable et représentatif, par exemple sur la base de la dispersion de la vitesse, de la dispersion de la longueur de foulée, de la dispersion du temps de vol etc. Des tests ont cependant montré qu'il est particulièrement intéressant de déterminer un indice de régularité comme une fonction de la réactivité $T_v/T_c$, par exemple en calculant la dispersion de la réactivité.

Une course irrégulière est souvent un indice de fatigue ; le temps de vol tend à diminuer, et le temps de contact avec le sol à augmenter lorsque le coureur se fatigue. Il est donc possible de calculer un niveau de fatigue sur la base de la régularité, par exemple à l'aide de la formule :

$$Fatigue = 1 - \frac{\sum_{i=1}^{n} \text{Re}\,gularité(i)}{n \cdot \text{Re}\,gularité(1)}$$

Il est aussi possible de déterminer et de s'intéresser par exemple au travail mécanique vertical (dépendant de l'abaissement ou de l'ondulation du centre de gravité) et/ou au travail mécanique horizontal.

L'efficacité de la foulée peut aussi être affectée par une asymétrie entre chacune des jambes. Un niveau d'asymétrie peut par exemple être calculé en comparant ou en effectuant la différence entre au moins un paramètre de chacune des jambes mesuré avec l'accéléromètre. Il s'est avéré lors de tests particulièrement significatifs de déterminer la différence entre le temps de contact de chacune des jambes, selon la relation :

$$Asymétrie = (T_c\,max - T_c\,min)/T_c\,min$$

Il serait aussi possible de déterminer par exemple la différence entre le temps de vol de chacune des jambes.

Une asymétrie est parfois un indice précurseur d'un risque de blessure ou d'une blessure mal guérie. Il est donc possible de calculer un risque de blessure comme fonction de l'asymétrie, par exemple à l'aide de la relation :

$$Risque\ de\ blessure = f(Asymétrie) = R.Blessure = 1 - \frac{\sum_{i=1}^{n} Asy(i)}{n \cdot Asy_{max}}$$

L'ensemble des différents paramètres ci-dessus ne peut pas être déterminé avec les dispositifs de l'art antérieur; chaque paramètre individuel a sa propre signification, utile au sportif et à son entraîneur, et peut être déterminé indépendamment des autres paramètres.

Il est possible de mesurer ces paramètres sur l'ensemble d'un test, par exemple en calculant la valeur moyenne de chaque paramètre au cours d'un test de 400 mètres ou sur une autre longueur. Il est aussi possible de calculer une séquence de valeurs instantanées afin d'observer l'évolution de chaque paramètre au cours du test, par exemple la longueur de chaque foulée etc. Dans un autre mode de réalisation, la distance parcourue est automatiquement ou manuellement segmentée en différents tronçons et les paramètres correspondant à chaque tronçon sont calculés et restitués. Les tronçons peuvent par exemple correspondre à des distances prédéterminées, par exemple en décomposant une distance ou encore en paramétrant des mesures de quelques secondes à intervalles réguliers ou à un endroit précis du parcours en pressant volontairement sur le bouton du dispositif. Le dispositif permet de choisir les paramètres et le tronçon qui doivent être affichés.

Dans une variante, les tronçons correspondent à des segments avec des caractéristiques différentes, par exemple des segments de déclivité différente, ou des segments parcourus à des vitesses différentes. Des paramètres peuvent aussi être calculés sur la base de moyennes calculées pendant des segments discontinus et regroupés automatiquement, par exemple afin de calculer des moyennes de paramètres sur l'ensemble des segments de montée ou de descente. La segmentation peut aussi être effectuée automatiquement sur la base des données d'un récepteur satellitaire de type GPS par exemple.

Une mesure de paramètres biomécaniques peut être effectuée comme indiqué pendant un test sur une distance étalonnée, par exemple sur un tour de piste d'athlétisme. Il est aussi possible en complément d'effectuer une mesure et un affichage pendant des séances d'entraînement normales, par exemple pendant un jogging sur le terrain, pendant une compétition etc. Bien que les mesures soient perturbées par les chocs sur le terrain et par l'imprécision quant à la distance parcourue, ce mode de fonctionnement permet de mesurer les mêmes paramètres dans d'autres conditions, ou d'autres paramètres tels que par exemple l'évolution de la foulée au cours d'une séance d'entraînement de grande durée, ou lorsque l'athlète se déplace sur un sol irrégulier ou non plat. Il est par exemple possible de fournir des résultats différenciés pour une course au plat, à la montée ou à la descente. Cette inclinaison peut être déterminée par un récepteur satellitaire, par l'utilisateur lui-même, ou par interprétation des signaux produits par l'accéléromètre dans la direction verticale et/ou dans une autre direction.

Le dispositif affiche et/ou restitue des paramètres biomécaniques liés à la foulée immédiatement après l'exercice, ou même pendant l'exercice. Le coureur peut ainsi se rendre compte pendant respectivement après la course de l'évolution des paramètres. Par exemple, il peut apprendre qu'il est en train de réduire la longueur de la foulée, ou le déplacement horizontal du centre de masse à chaque foulée, ou que sa raideur musculaire (« stiffness ») diminue, ce qui peut être un signe avant-coureur ou indicatif de fatigue musculaire.

Des tests ont montré que le dispositif permet de mesurer et d'évaluer les paramètres ci-dessus avec une précision significative et des valeurs de dispersion faibles, en employant les formules ci-dessus. Il est donc possible de déterminer des programmes d'entraînement spécifiques visant à améliorer à la fois les qualités techniques et les capacités physiques du coureur, tout en prévenant les blessures et risques de fatigue et en améliorant l'efficacité de la foulée.

Le dispositif permet avantageusement de générer des plans d'entraînement musculaires basés sur les paramètres mesurés et permettant d'améliorer le style de course et l'efficacité des foulées. Par exemple, le dispositif peut proposer des exercices de gymnastique, de renforcement musculaire ou d'assouplissement afin de renforcer certains paramètres, de façon personnalisée pour chaque coureur. Les exercices et plans d'entraînement proposés peuvent aussi comporter des séances de course à pied personnalisées, à un rythme et/ou avec des indications fournies visuellement et/ou auditivement par le dispositif.

## Revendications

1. Procédé mettant en oeuvre un accéléromètre (17) pour analyser des paramètres biomécaniques de la foulée d'un coureur de course à pied, comportant :

   a) fixation d'un dispositif (1) sur le tronc du coureur près du centre de gravité dudit coureur, ledit dispositif étant autonome électriquement et comportant un accéléromètre triaxial (17), un chronographe (16), un processeur numérique (19) ;
   b) une séquence de données d'accélération selon au moins la direction verticale ($a_v$) est mesurée par ledit accéléromètre pendant que le coureur parcourt une distance (D) sur un parcours de course ;
   c) pendant ou au terme de la course, des paramètres biomécaniques de la foulée sont calculés par ledit processeur sur la base desdites données d'accélération,

   **caractérisé en ce que**
   lesdits paramètres comprenant un indice de régularité de la foulée calculé sur la base de la dispersion de la réactivité ($T_v/T_c$) déterminée au moyen desdites données d'accélération, la réactivité étant calculée comme le rapport entre le temps de vol ($T_v$) et le temps de contact ($T_c$).

2. Le procédé selon la revendication 1, dans lequel lesdits paramètres biomécaniques incluent un indicateur de niveau de fatigue calculé sur la base dudit indice de régularité.

3. Le procédé selon la revendication précédente, dans lequel l'indicateur de fatigue est calculé à l'aide de la formule suivante :

$$Fatigue = 1 - \frac{\sum_{i=1}^{n} Régularité(i)}{n \cdot Régularité(1)}$$

dans laquelle

   *Fatigue* indique l'indicateur de fatigue
   *Régularité* indique l'indice de régularité
   n indique le nombre des foulées.

4. Le procédé selon l'une des revendications précédentes, ladite distance (D) étant soit prédéterminée, soit introduite manuellement, soit déterminée par un récepteur satellitaire.

5. Le procédé de l'une des revendications précédentes, dans lequel une séquence de données d'accélération selon au moins la direction antéropostérieure est mesurée par ledit accéléromètre (17) et traitée séparément de ladite séquence de données d'accélération selon la direction verticale afin de calculer au moins certains desdits paramètres.

6. Le procédé de l'une des revendications précédentes, dans lequel une séquence de données d'accélération selon au moins la direction latérale est mesurée par ledit accéléromètre et traitée séparément de ladite séquence de données d'accélération selon la direction verticale afin de calculer au moins certains desdits paramètres.

7. Le procédé de l'une des revendications précédentes, dans lequel la direction verticale est déterminée sur la base des données d'accélération lorsque le coureur est à l'arrêt.

8. Le procédé de l'une des revendications 5 à 7, dans lequel la direction antéropostérieure est déterminée en déterminant la direction spatiale dans lequel le déplacement est le plus important.

9. Le procédé de l'une des revendications 7 ou 8 dans lequel une transformation de référentiel est effectuée lorsque les axes dudit accéléromètre ne coïncident pas avec lesdits axes verticaux respectivement antéropostérieurs.

10. Support de données informatique stockant de manière semi-permanente un programme informatique destiné à être exécuté par un processeur numérique (19) du dispositif selon la revendication 11 afin d'exécuter le procédé de l'une des revendications 1 à 9.

11. Dispositif destiné à l'analyse des paramètres biomécaniques de la foulée d'un coureur de course à pied et comportant :

   une source d'alimentation électrique autonome (18) ;
   un accéléromètre triaxial (17) apte à fournir au moins une séquence de données d'accélération ($a_v$) selon au moins la direction verticale pendant que le coureur parcourt une distance sur un parcours de course;
   un chronographe (16) ;
   une ceinture (15) ;
   un processeur numérique (19) programmé afin de calculer, pendant ou au terme de la course, des paramètres biomécaniques de la foulée dudit coureur, sur la base desdites données d'accélération, de ladite distance et d'une durée comptée par ledit chronographe,

   **caractérisé en ce que**
   lesdits paramètres biomécaniques comprenant un indice de régularité de la foulée, calculé sur la base de la dispersion de la réactivité ($T_v/T_c$), la réactivité étant calculée comme le rapport entre le temps de vol ($T_v$) et le temps de contact ($T_c$).

**Patentansprüche**

1. Verfahren unter Verwendung eines Beschleunigungsmessers (17) zur Analyse der biomechanischen Parameter des Schritts eines Läufers beim Laufen, umfassend:

   a) Befestigen einer Vorrichtung (1) am Rumpf des Läufers in der Nähe des Schwerpunkts des Läufers, wobei die Vorrichtung elektrisch autark ist und einen dreiachsigen Beschleunigungsmesser (17), einen Chronographen (16) und einen digitalen Prozessor (19) umfasst;
   b) eine Folge von Beschleunigungsdaten gemäß mindestens der vertikalen Richtung ($a_v$) von dem Beschleunigungsmesser gemessen wird, während der Läufer eine Distanz (D) auf einer Laufstrecke zurücklegt;
   c) während oder am Ende des Laufs werden biomechanische Parameter des Schritts von dem Prozessor auf der Grundlage der Beschleunigungsdaten ermittelt, **dadurch gekennzeichnet, dass**

   die Parameter einen Gleichmäßigkeitsindex des Schritts umfassen, der auf der Grundlage der Streuung der Reaktionsfähigkeit ($T_v/T_c$) berechnet wird, die mittels der Beschleunigungsdaten bestimmt wird, wobei die Reaktionsfähigkeit als das Verhältnis zwischen der Flugzeit ($T_v$) und der Kontaktzeit ($T_c$) berechnet wird.

2. Das Verfahren nach Anspruch 1, wobei die biomechanischen Parameter einen Indikator für den Grad der Ermüdung beinhalten, der auf der Basis des Gleichmäßigkeitsindexes berechnet wird.

3. Das Verfahren nach dem vorstehenden Anspruch, bei dem der Grad der Ermüdung mithilfe der folgenden Formel berechnet wird:

$$Fatigue = 1 - \frac{\sum_{i=1}^{n} Régularité(i)}{n \cdot Régularité(1)}$$

   wobei

   *Fatigue* bezeichnet den Grad der Ermüdung;
   *Régularité* bezeichnet den Gleichmäßigkeitsindex; und
   n bezeichnet die Anzahl der Schritte.

4. Das Verfahren nach einem der vorhergehenden Ansprüche, wobei die Distanz (D) entweder vorbestimmt, manuell eingegeben oder von einem Satellitenempfänger bestimmt wird.

5. Das Verfahren nach einem der vorstehenden Ansprüche, bei dem eine Folge von Beschleunigungsdaten in mindestens der anterioren Richtung von dem Beschleunigungsmesser (17) erfasst und getrennt von der Folge von Beschleunigungsdaten in der vertikalen Richtung verarbeitet wird, um mindestens einige der Parameter zu berechnen.

6. Das Verfahren nach einem der vorstehenden Ansprüche, bei dem eine Folge von Beschleunigungsdaten zumindest in lateraler Richtung von dem Beschleunigungsmesser erfasst und getrennt von der Folge von Beschleunigungsdaten in vertikaler Richtung verarbeitet wird, um zumindest einige der Parameter zu berechnen.

7. Das Verfahren nach einem der vorstehenden Ansprüche, wobei die vertikale Richtung auf der Grundlage der Beschleunigungsdaten ermittelt wird, wenn der Läufer sich im Stillstand befindet.

8. Das Verfahren nach einem der Ansprüche 5 bis 7, wobei die anterior-posteriore Richtung durch Bestimmung der Raumrichtung ermittelt wird, in der die Auslenkung am größten ist.

9. Das Verfahren nach einem der Ansprüche 7 oder 8, bei dem eine Transformation des Bezugssystems durchgeführt wird, falls die Achsen des Beschleunigungsmessers nicht mit den vertikalen bzw. anteroposterioren Achsen übereinstimmen.

10. Computerdatenträger, der ein Softwareprogramm halbpermanent speichert, das vom digitalen Prozessor (19) des Geräts nach Anspruch 11 ausgeführt werden soll, um das Verfahren nach einem der Ansprüche 1 bis 9 auszuführen.

**11.** Vorrichtung zur Analyse der biomechanischen Parameter des Schritts eines Läufers beim Laufen, mit:

einer elektrisch autarken Stromquelle (18);
einem dreiachsigen Beschleunigungsmesser (17), der so ausgebildet ist, dass er mindestens eine Folge von Beschleunigungsdaten ($a_v$) in mindestens der vertikalen Richtung bereitstellt, während der Läufer eine Distanz auf einer Laufstrecke zurücklegt;
einem Chronographen (16) ;
einem Gürtel (15) ;
einem digitalen Prozessor (19), der so konfiguriert ist, dass er während oder am Ende des Laufs biomechanische Parameter des Schritts des Läufers auf der Grundlage der Beschleunigungsdaten, der Distanz und einer von dem Chronographen erfassten Zeit berechnet, **dadurch gekennzeichnet, dass** die biomechanischen Parameter einen Gleichmäßigkeitsindex des Schritts umfassen, der auf der Grundlage der Streuung der Reaktionsfähigkeit ($T_v/T_c$) berechnet wird, wobei die Reaktionsfähigkeit als das Verhältnis zwischen der Flugzeit ($T_v$) und der Kontaktzeit ($T_c$) berechnet wird.

**Claims**

**1.** Method using an accelerometer (17) to analyze biomechanical parameters of the stride of a runner, comprising:

a) fixing a device (1) to the trunk of the runner near the centre of gravity of said runner, said device being electrically autonomous and comprising a triaxial accelerometer (17), a chronograph (16), a digital processor (19);
b) a sequence of acceleration data in at least the vertical direction ($a_v$) is measured by said accelerometer while the runner is covering a distance (D) on a race course;
c) during or at the end of the run, biomechanical parameters of the stride are calculated by said processor on the basis of said acceleration data

**characterized in that**
said parameters comprising a stride regularity index calculated on the basis of the dispersion of the reactivity ($T_v/T_c$) determined by means of said acceleration data, the reactivity being calculated as the ratio between the time of flight ($T_v$) and the contact time ($T_c$).

**2.** The method according to claim 1, wherein said biomechanical parameters include a fatigue level indicator calculated on the basis of said regularity index.

**3.** The method according to the preceding claim, wherein the fatigue indicator is calculated using the following formula:

$$Fatigue = 1 - \frac{\sum_{i=1}^{n} Régularité(i)}{n \cdot Régularité(1)}$$

in which

*Fatigue* indicates the fatigue indicator
*Régularité* indicates the regularity index
n indicates the number of strides.

**4.** The method according to any of the preceding claims, said distance (D) being predetermined, manually entered or determined by a satellite receiver.

**5.** The method of any of the preceding claims, wherein a sequence of acceleration data according to at least the anteroposterior direction is measured by said accelerometer (17) and processed separately from said sequence of acceleration data according to the vertical direction, in order to calculate at least some of said parameters.

6. The method of any of the preceding claims, wherein a sequence of acceleration data along at least the lateral direction is measured by said accelerometer and processed separately from said sequence of acceleration data along the vertical direction, to calculate at least some of said parameters.

7. The method of any of the preceding claims, wherein the vertical direction is determined on the basis of the acceleration data when the runner is stationary.

8. The method of any of claims 5 to 7, wherein the anteroposterior direction is determined by determining the spatial direction in which the displacement is greatest.

9. The method of any of claims 7 or 8 wherein a reference frame transformation is performed when the axes of said accelerometer do not coincide with said vertical respectively anteroposterior axes.

10. Computer data carrier semi-permanently storing a computer program for execution by a digital processor (19) of the device according to claim 11 in order to perform the method of any of claims 1 to 9.

11. Device for analyzing the biomechanical parameters of the stride of a runner and comprising:

    a self-contained electrical power source (18);
    a triaxial accelerometer (17) capable of providing at least one sequence of acceleration data ($a_v$) in at least the vertical direction while the runner covers a distance on a running course;
    a chronograph (16);
    a belt (15);
    a digital processor (19) programmed to calculate, during or at the end of the race, biomechanical parameters of the stride of said runner, on the basis of said acceleration data, said distance and a time counted by said chronograph,

    **characterized in that**
    said biomechanical parameters comprising a stride regularity index, calculated on the basis of the dispersion of the reactivity ($T_v/T_c$), the reactivity being calculated as the ratio between the flight time ($T_v$) and the contact time ($T_c$).

Fig. 1

Fig. 2

Fig. 3

Fig. 4

Fig. 5

Fig. 6

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1862765 A
- US 6397151 B
- DE 4426302
- WO 09094746 A
- EP 1897598 A
- WO 2009094746 A
- US 6148280 A
- EP 418548 A
- US 5474083 A
- WO 07107491 A
- EP 2027817 A
- WO 2010046448 A
- WO 2007036611 A
- US 2009018794 A
- EP 1754521 A
- WO 2007017471 A

- US 5788655 A
- WO 2005074795 A
- WO 03032826 A
- EP 1992389 A
- US 5056783 A
- US 20080288200 A
- EP 1253404 A
- US 5976083 A
- WO 2006030065 A
- EP 1764583 A
- WO 2005091113 A
- US 20080190202 A
- WO 2007102134 A
- GB 2415788 A
- US 20080214360 A1